(19) Europäisches Patentamt
European Patent Office
Office européen des brevets

(11) EP 3 250 151 B1

(12) FASCICULE DE BREVET EUROPEEN

(45) Date de publication et mention de la délivrance du brevet:
**10.03.2021 Bulletin 2021/10**

(51) Int Cl.:
*A61F 2/14* (2006.01)          *A61F 9/007* (2006.01)
*A61N 1/05* (2006.01)

(21) Numéro de dépôt: **16705238.0**

(22) Date de dépôt: **26.01.2016**

(86) Numéro de dépôt international:
**PCT/FR2016/050156**

(87) Numéro de publication internationale:
**WO 2016/120557 (04.08.2016 Gazette 2016/31)**

(54) **DISPOSITIF DE REHABILITATION PROTHETIQUE DE LA RETINE**

VORRICHTUNG FÜR DIE PROTHETISCHE REHABILITATION DER NETZHAUT

DEVICE FOR PROSTHETIC REHABILITATION OF THE RETINA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.01.2015 FR 1550639**

(43) Date de publication de la demande:
**06.12.2017 Bulletin 2017/49**

(73) Titulaire: **Commissariat à l'Énergie Atomique et aux Énergies Alternatives 75015 Paris (FR)**

(72) Inventeurs:
• PANNETIER-LECOEUR, Myriam
91440 Bures-sur-Yvette (FR)
• TRAUCHESSEC, Vincent
92160 Antony (FR)
• CARUSO, Laure
75013 Paris (FR)
• CHAVANE, Frederic
13004 Marseille (FR)
• ROUX, Sebastien
13600 la Ciotat (FR)
• MATONTI, Frederic
13005 Marseille (FR)

(74) Mandataire: **Cabinet Camus Lebkiri 25 rue de Maubeuge 75009 Paris (FR)**

(56) Documents cités:
WO-A1-00/74600      WO-A1-2013/126498
ES-A1- 2 370 014     FR-A1- 2 673 481

## Description

## DOMAINE TECHNIQUE DE L'INVENTION

Le domaine technique de l'invention est celui de la réhabilitation prothétique de la rétine. La présente invention concerne un dispositif de réhabilitation prothétique de la rétine. Une application importante de l'invention concerne le traitement de maladies de la rétine de type rétinopathies dégénératives, ou dégénérescences maculaires liées à l'âge (DMLA).

## ARRIERE-PLAN TECHNOLOGIQUE DE L'INVENTION

Les dégénérescences rétiniennes, telles que les dégénérescences maculaires liées à l'âge (DMLA) et les rétinites pigmentaires, concernent actuellement une large partie de la population mondiale, proportion qui s'accroit avec le vieillissement de la population.

Une piste explorée pour soigner ces pathologies est de réaliser une stimulation artificielle des neurones ganglionnaires dans le but de transférer à travers le nerf optique un signal utilisable par le système visuel.

Une telle stimulation ou activation neuronale peut être artificiellement induite par des moyens pharmacologiques ou par excitation électrique directe en appliquant une différence de potentiels sur le neurone ou l'ensemble de neurones à exciter.

Dans le cas d'une stimulation par moyens pharmacologiques, la réponse et le temps de récupération peuvent être assez lents, en raison du temps de diffusion de l'agent pharmacologique jusqu'à la zone d'intérêt. A l'inverse, la stimulation électrique est très rapide, mais nécessite un contact électrique avec le neurone ou la zone à exciter. Le contact électrique peut se faire par contact physique d'un conducteur avec le neurone ou la zone à exciter proprement dits, ou en plaçant un conducteur au voisinage immédiat du neurone ou de la zone à exciter, compte tenu de la conductivité des tissus qui environnent le neurone ou la zone à exciter. On connait ainsi des dispositifs de rétines artificielles électriques qui comportent des systèmes de microélectrodes implantés au contact de la région à exciter. Des impulsions électriques sont délivrées au moyen d'un générateur externe sur un nombre de microélectrodes typiquement compris entre quelques dizaines et quelques centaines.

Néanmoins, cette technique présente certaines limitations. Une première limitation est due à la diffusion du courant électrique dans les tissus alentours à la région ciblée. En particulier, des neurones non ciblés par la stimulation peuvent être activés via leur axone passant dans la région stimulée. La sélectivité spatiale de l'excitation et l'efficacité de l'excitation sur une zone focalisée sont ainsi fortement limitées.

Une seconde limitation est liée à la nécessité de positionner l'implant au plus près des neurones à exciter, ce qui implique une chirurgie complexe et risquée d'implantation. En effet, quel que soit le type de prothèse employé, leur positionnement doit se faire au contact direct de la rétine, ce qui impose une chirurgie sous- ou épi-rétinienne potentiellement délabrante. Ce mode d'implantation au contact direct de la rétine limite également les dimensions de l'implant à des dimensions latérales de l'ordre de 3 mm de côté si l'implant présente une surface carrée, ou de 3 mm de diamètre si l'implant présente une surface ronde. A de telles dimensions, la déformation due à la courbure de l'œil est très faible, mais l'implant ne peut donc activer qu'une portion très limitée de la surface totale de la rétine. Enfin, la réaction des tissus à la présence de l'implant peut entrainer à moyen et long terme le développement, par une réaction inflammatoire et de cicatrisation, de tissus de granulation qui, en recouvrant les microélectrodes, diminuent fortement leur efficacité en isolant l'interface microélectrode-tissu. Ce problème est commun aux implants reposant sur la stimulation électrique, tels que les implants de stimulation cérébrale profonde, ou les implants corticaux dans le cadre d'interfaces cerveau-machine par exemple.

Il est également connu d'induire une activation neuronale en utilisant une impulsion de champ magnétique. En effet, une variation rapide de champ magnétique, créée par exemple par une impulsion de courant dans une bobine, génère un champ électrique suivant la loi de Faraday :

$$\nabla \times E = -\frac{\partial B}{\partial t}.$$

L'intérêt de l'utilisation de l'impulsion de champ magnétique sur un ou plusieurs neurones, en termes d'application clinique, est lié à la possibilité de stimulation à distance des neurones. Ainsi, suivant la géométrie du bobinage, il est possible de créer une zone d'activation à distance de la bobine excitatrice, et d'activer ou d'inhiber des neurones sans contact physique avec ceux-ci, ni même avec les tissus les entourant.

A un niveau macroscopique, ce principe est utilisé pour stimuler des populations de neurones à l'échelle des zones corticales du cerveau ; elle est désignée sous l'appellation de Stimulation Magnétique Transcranienne. Cette technique utilise de grosses bobines avec une dimension latérale de 10 à 30 centimètres, dans lesquelles une impulsion de courant est appliquée, entrainant une variation rapide du champ magnétique et donc la création d'un champ électrique sur une zone du cerveau sélectionnée par l'emplacement et la géométrie des bobines. Les bobines utilisées en stimulation magnétique transcranienne sont typiquement des bobines planaires multispires, avec généralement entre 10 et 30 spires, de forme circulaire, carrée ou rectangulaire. Les dimensions d'une telle bobine sont typiquement de l'ordre de 5 à 15 cm de côté ou de diamètre. Deux bobines adjacentes sont généralement utilisées dans les techniques de stimulation magnétique transcranienne. Il a été montré que cette technique permet soit d'activer soit d'inhiber une population neuronale, en fonction de la polarité du champ E par rapport à la population. Cette technique macroscopique est utilisée dans le cadre de recherches en sciences cognitives sur des cerveaux sains ou pathologiques, ainsi que dans le traitement de désordres psychiatriques, par exemple sur des dépressions résistantes aux traitements médicamenteux.

Le document « Functional Magnetic Stimulation for Implantable Epiretinal Prosthesis », par E. Basham, M. Sivaprakasam et W. Liu (2005) suggère, afin de restaurer la vision de patients atteints de rétinites pigmentaires ou de dégénérescences maculaires liées à l'âge, d'utiliser la stimulation magnétique en pointant les avantages en termes de biocompatibilité et de biorésistance par rapport à la stimulation électrique. Toutefois, le problème de la complexité de la chirurgie d'implantation de tels implants demeure entier. Un dispositif selon le préambule de la revendication 1 est connu du document ES-A-2 370 014.

## RESUME DE L'INVENTION

L'invention offre une solution aux problèmes évoqués précédemment en proposant un dispositif de réhabilitation prothétique de la vision permettant une chirurgie d'implantation simplifiée par rapport à l'état de la technique connu.

Un aspect de l'invention concerne donc un dispositif de réhabilitation prothétique de la vision comportant :

- un explant scléral dont la forme est adaptée pour être au contact d'au moins une partie de la sclère d'un œil, et
- au moins un inducteur agencé sur l'explant scléral, l'explant scléral comportant un substrat à la surface duquel est agencé l'inducteur, et une couche isolante déposée sur l'inducteur et sur la partie du substrat qui n'est pas recouverte par l'inducteur; l'inducteur créant un champ électromagnétique de stimulation d'une zone d'excitation et la couche isolante isolant galvaniquement l'inducteur de ladite zone d'excitation. L'invention permet ainsi avantageusement une stimulation d'une zone d'excitation sans irritation ni réaction inflammatoire ou de cicatrisation de ladite zone d'excitation, et donc le rallongement de la durée de vie de l'explant.

Grâce à l'invention, on utilise un explant scléral comportant au moins un inducteur pour permettre une excitation à distance de neurones ganglionnaires, l'explant scléral pouvant être agencé sur la sclère d'un œil grâce à une chirurgie d'implantation simplifiée, plus rapide et moins risquée que dans l'état de la technique. Le dispositif de réhabilitation prothétique de la vision selon un aspect de l'invention présente ainsi l'avantage d'être moins invasif que les dispositifs de l'état de la technique.

Outre les caractéristiques qui viennent d'être évoquées dans le paragraphe précédent, le dispositif de réhabilitation prothétique selon un aspect de l'invention peut présenter une ou plusieurs caractéristiques complémentaires parmi les suivantes, considérées individuellement ou selon toutes les combinaisons techniquement possibles :

- L'explant scléral présente une forme de calotte sphérique. L'explant scléral est ainsi avantageusement apte à être agencé au contact d'au moins une partie de la sclère d'un œil. L'explant scléral de l'invention permet avantageusement de couvrir une zone rétinienne beaucoup plus étendue que lors de l'utilisation d'un implant sous-rétinien ou épi-rétinien. En particulier, la calotte sphérique peut être un hémisphère.

- L'explant scléral comporte une partie centrale et une partie périphérique entourant la partie centrale, et le au moins un inducteur est agencé sur la partie périphérique de l'explant scléral.

- Selon un premier mode de réalisation de l'invention, l'explant scléral comporte une ouverture. L'explant scléral selon le premier mode de réalisation de l'invention peut ainsi avantageusement être agencé en arrière d'un œil, le passage du nerf optique de l'œil étant prévu à travers l'ouverture. L'ouverture est avantageusement agencée dans la partie centrale de l'explant scléral.

- Selon le premier mode de réalisation de l'invention, l'explant scléral comporte une fente agencée entre une périphérie extérieure de l'explant scléral et l'ouverture de l'explant scléral, la fente définissant deux extrémités de l'explant scléral pouvant être écartées l'une de l'autre. La fente de l'explant scléral contribue avantageusement à faciliter l'agencement de l'explant scléral sur un œil, particulièrement lors d'une configuration de l'explant scléral en arrière d'un œil. En effet, en écartant les deux extrémités de l'explant scléral, de part et d'autre de la fente, on permet le passage d'un nerf optique d'un œil dont on souhaite restaurer la vision.

- Selon une variante du premier mode de réalisation de l'invention, l'explant scléral présente un système d'accroche des deux extrémités de l'explant scléral. Le système d'accroché de l'explant scléral permet avantageusement de refermer l'explant scléral, typiquement après avoir fait passer l'explant scléral sous les muscles extra-oculaires et permis le passage du nerf optique dans l'ouverture de l'explant scléral.

- Le dispositif de réhabilitation prothétique de la vision comporte une pluralité d'inducteurs agencée sur l'explant scléral. Le positionnement et l'écartement de chaque inducteur vis-à-vis des autres inducteurs de la pluralité d'inducteurs est avantageusement calculé pour assurer l'indépendance de chaque inducteur.

Un autre aspect de l'invention concerne un système de réhabilitation prothétique de la vision comportant :

- une unité d'acquisition d'images ;
- une unité de traitement d'images, recevant en entrée une image acquise par le système d'acquisition d'images et renvoyant en sortie un codage de ladite image ;
- une unité de commande, recevant en entrée le codage de ladite image et renvoyant en sortie une commande d'un générateur d'impulsions ;
- le générateur d'impulsions, envoyant selon la commande de l'unité de commande au moins une impulsion vers un multiplexeur ;
- le multiplexeur, transmettant la au moins une impulsion vers le au moins un inducteur ou vers au moins une partie de la pluralité d'inducteurs d'un dispositif de réhabilitation prothétique de la vision selon un aspect de l'invention.

L'invention et ses différentes applications seront mieux comprises à la lecture de la description qui suit et à l'examen des figures qui l'accompagnent.

**BREVE DESCRIPTION DES FIGURES**

Les figures sont présentées à titre indicatif et nullement limitatif de l'invention.

- La figure 1a montre schématiquement une première vue d'un dispositif de réhabilitation prothétique de la vision selon un premier mode de réalisation de l'invention.
- La figure 1b montre schématiquement une deuxième vue du dispositif de réhabilitation prothétique de la vision selon le premier mode de réalisation de l'invention.
- La figure 1c montre schématiquement une vue du dispositif de réhabilitation de la vision selon une variante du premier mode de réalisation de l'invention.
- La figure 2 montre schématiquement différents paramètres de dimensionnement du dispositif de réhabilitation prothétique de la vision selon le premier mode de réalisation de l'invention.
- La figure 3 montre schématiquement une vue partielle en coupe d'un dispositif de réhabilitation prothétique de la vision selon un aspect de l'invention.
- La figure 4a montre schématiquement une première vue du dispositif de réhabilitation prothétique de la vision selon le premier mode de réalisation de l'invention, agencé au contact d'une partie de la sclère d'un œil.
- La figure 4b montre schématiquement une deuxième vue du dispositif de réhabilitation prothétique de la vision selon le premier mode de réalisation de l'invention, agencé au contact d'une partie de la sclère d'un œil.
- La figure 5 schématise le principe physique d'activation neuronale par une impulsion électromagnétique d'un dispositif de réhabilitation prothétique de la vision selon un aspect de l'invention.
- La figure 6a montre schématiquement le fonctionnement d'un dispositif de réhabilitation prothétique de la vision selon un aspect de l'invention, comportant un unique inducteur.
- La figure 6b montre schématiquement le fonctionnement d'un dispositif de réhabilitation prothétique de la vision selon un aspect de l'invention, comportant des premier et deuxième inducteurs.
- La figure 7a montre schématiquement le point focal de champ magnétique obtenu avec une première configuration d'une matrice d'inducteurs.
- La figure 7b montre schématiquement le point focal de champ magnétique obtenu avec une deuxième configuration de la matrice d'inducteurs de la figure 7a.
- La figure 7c montre schématiquement le point focal de champ magnétique obtenu avec une troisième configuration de la matrice d'inducteurs de la figure 7a.
- La figure 8a montre schématiquement une première vue d'un dispositif de réhabilitation prothétique de la vision selon un deuxième mode de réalisation de l'invention.
- La figure 8b montre schématiquement une deuxième vue du dispositif de réhabilitation prothétique de la vision selon le deuxième mode de réalisation de l'invention.
- La figure 9 montre schématiquement une vue du dispositif de réhabilitation prothétique de la vision selon le deuxième mode de réalisation de l'invention, agencé au contact d'une partie de la sclère d'un œil.
- La figure 10 montre une représentation schématique d'un système de réhabilitation prothétique de la vision selon un aspect de l'invention, utilisant un dispositif de réhabilitation prothétique de la vision selon le premier ou le deuxième mode de réalisation de l'invention.

**DESCRIPTION DETAILLEE D'AU MOINS UN MODE DE REALISATION DE L'INVENTION**

Sauf précision contraire, un même élément apparaissant sur des figures différentes présente une référence unique.

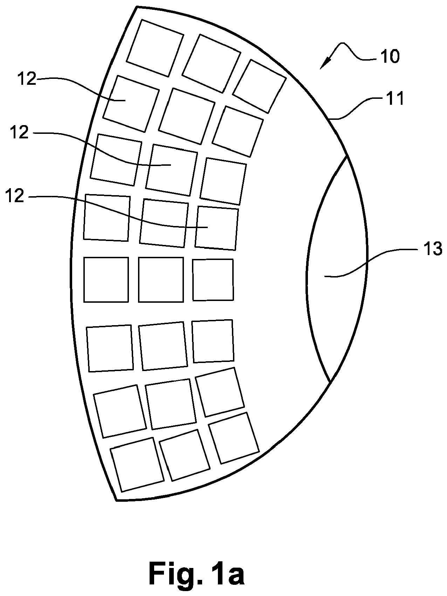

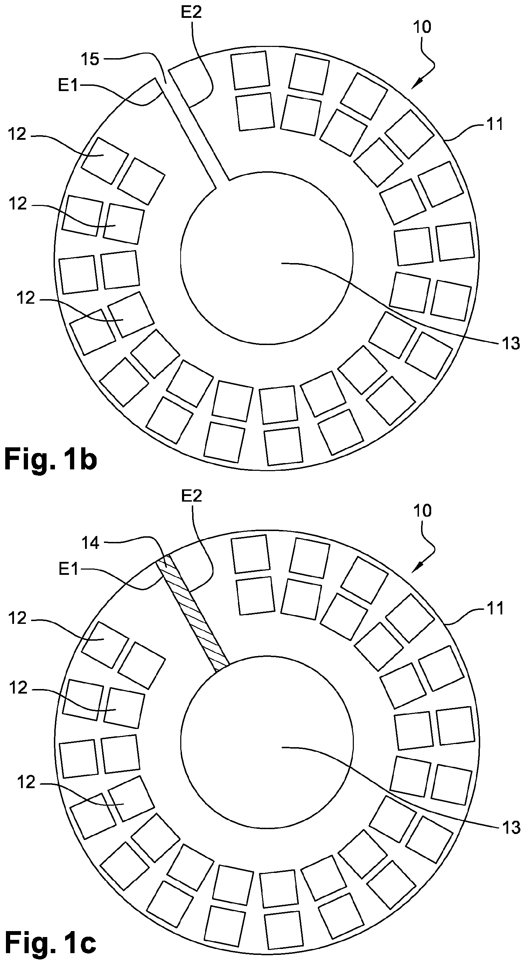

La figure 1a montre schématiquement une première vue, de côté, d'un dispositif 10 de réhabilitation prothétique de la vision selon un premier mode de réalisation de l'invention. La figure 1b montre schématiquement une deuxième vue, de dos, du dispositif 10 de réhabilitation prothétique de la vision selon le premier mode de réalisation de l'invention. La figure 1c montre schématiquement une vue de dos d'une variante du dispositif 10 de réhabilitation prothétique de la vision selon le premier mode de réalisation de l'invention, selon laquelle le dispositif 10 comporte un système d'accroché 14. Les figures 1a, 1b et 1c sont décrites conjointement.

Le dispositif 10 de réhabilitation prothétique de la vision selon le premier mode de réalisation de l'invention comporte :

- un explant scléral 11 dont la forme est adaptée pour être au contact d'au moins une partie de la sclère d'un œil, et
- une pluralité d'inducteurs 12 agencés sur l'explant scléral 11.

La sclère est une membrane protectrice de l'œil. Selon le premier mode de réalisation de l'invention, l'explant scléral 11 comporte avantageusement une ouverture 13. L'ouverture 13 est dimensionnée pour permettre le passage d'un nerf optique. L'explant scléral 11 est ainsi adapté pour être au contact d'au moins une partie de la sclère d'un œil, le nerf optique de l'œil passant par l'ouverture 13. Selon le premier mode de réalisation de l'invention, l'explant scléral 11 comporte avantageusement une fente 15, ou coupure, réalisée entre la périphérie extérieure de l'explant scléral 11 et l'ouverture 13 de l'explant scléral 11. La fente 15 définit une première extrémité E1 et une deuxième extrémité E2 de l'explant scléral 11, de part et d'autre de la fente 15. Les première et deuxième extrémités E1 et E2 de l'explant scléral 11 peuvent être écartées l'une de l'autre pour permettre de glisser l'explant scléral 11 sous les muscles extra-oculaires d'un œil, et pour permettre le passage d'un nerf optique et l'agencement du nerf optique dans l'ouverture 13 de l'explant scléral 11. La fente 15 permet donc avantageusement de faciliter la mise en place de l'explant scléral 11 en face arrière d'un œil dont on souhaite restaurer la vision. Lorsqu'aucune contrainte n'est exercée sur elles, les première et deuxième extrémités E1 et E2 de l'explant scléral 11 se trouvent typiquement en face l'une de l'autre, alignées l'une avec l'autre. Le système d'accroché 14 selon la variante du premier mode de réalisation de l'invention représentée à la figure 1c permet avantageusement d'accrocher les première et deuxième extrémités E1 et E2 l'une avec l'autre de manière à empêcher qu'elles ne s'écartent l'une de l'autre. Ainsi, typiquement après avoir écarté les première et deuxième extrémités E1 et E2 de l'explant scléral 11 l'une de l'autre pour permettre le passage du nerf optique, l'explant scléral 11 peut être refermé de manière robuste.

L'explant scléral 11 selon le premier mode de réalisation de l'invention est destiné à être agencé sur la sclère recouvrant la face externe de l'œil, en arrière de l'œil. Un tel positionnement de l'explant scléral 11 peut être obtenu grâce à une chirurgie simple et peu invasive consistant à insérer l'explant scléral 11 à travers une incision réalisée dans la conjonctive. Un exemple particulier de mode opératoire chirurgical sera détaillé plus loin dans le présent document. Un avantage important du premier mode de réalisation de l'invention est de permettre une grande couverture de la rétine et une excitation de neurones sur une surface maximale, couvrant un large champ visuel, contrairement aux dispositifs selon l'état de la technique qui n'utilisent qu'une faible zone de stimulation.

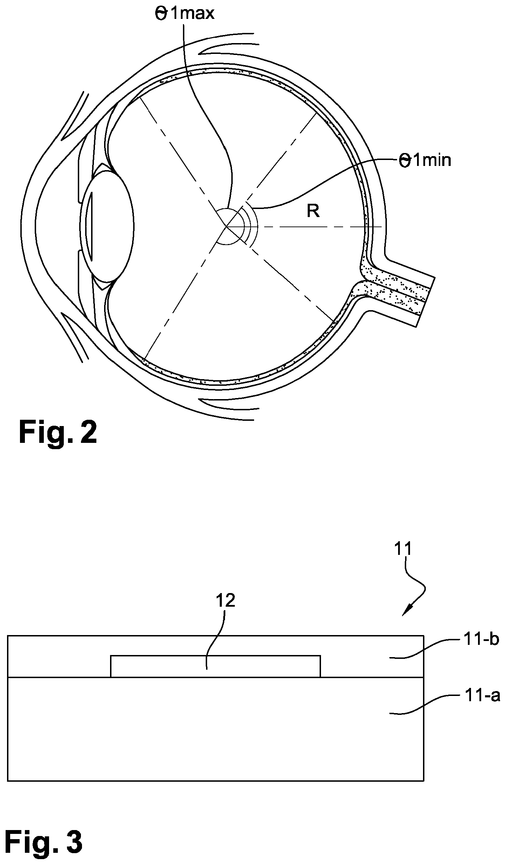

Dans l'exemple particulier illustré aux figures 1a à 1c, l'explant scléral 11 présente ainsi une forme de lentille percée, adaptée à la courbure naturelle de la sclère. L'explant scléral 11 peut également être décrit comme étant une calotte sphérique, dont les dimensions sont choisies pour permettre l'adaptation de l'explant scléral 11 sur un œil. Un œil pouvant typiquement être assimilé à une sphère ayant un diamètre de l'ordre de 23,5 mm, la calotte sphérique est préférentiellement définie à partir d'une sphère S ayant un rayon R compris entre 10 mm et 14 mm et typiquement égal à 12 mm, la calotte sphérique étant limitée par l'intersection avec la sphère S d'un premier cône de révolution C1 de sommet le centre de la sphère S et de demi-angle au sommet $\theta 1$ compris entre 45° et 110°. En particulier, la calotte sphérique peut être un hémisphère. La figure 2 montre schématiquement un globe oculaire et illustre la manière dont les paramètres R et $\theta 1$ sont définis.

Compte-tenu des dimensions d'un nerf optique et de son emplacement sur un globe oculaire, l'ouverture 13 présente avantageusement un diamètre compris entre 2 mm et 6 mm, et préférentiellement un diamètre de 3 mm. Il s'agit d'une manière générale de permettre le passage du nerf optique à travers l'ouverture 13 sans contact ou frottement entre le nerf optique et l'explant scléral, tout en ayant une couverture maximale de la région fovéale, la fovéa étant typiquement située à environ 2 mm du nerf optique. Dans l'exemple particulier où l'ouverture 13 présente un diamètre de 10 mm, l'ouverture 13 est avantageusement définie par un deuxième cône de révolution C2 de même génératrice que le premier cône de révolution C1 et de sommet le centre de la sphère S. Dans l'exemple particulier où l'ouverture 13 présente un diamètre de 2 mm, l'ouverture 13 est avantageusement définie en fonction du positionnement précis du nerf optique du globe oculaire.

La figure 3 montre schématiquement une vue partielle en coupe d'un dispositif 10 de réhabilitation prothétique de la vision selon un aspect de l'invention. La figure 3 montre en particulier que l'explant scléral 11 comporte :

- un substrat 11-a, à la surface duquel est agencé un inducteur 12, et
- une couche isolante 11-b, qui est déposée sur l'inducteur 12 et sur la partie du substrat 11-a qui n'est pas recouverte par l'inducteur 12.

Le substrat 11-a est réalisé en un matériau isolant, comme par exemple du kapton, du verre, du polyimide, du parylène, de la silicone ou du silico-hydrogel. L'inducteur 12 est réalisé en un matériau conducteur, comme par exemple du cuivre, de l'or ou un assemblage de plusieurs matériaux. L'inducteur 12 est préférentiellement réalisé en cuivre, qui présente une grande conductivité, éventuellement recouvert par une couche d'or ou de tantale, par exemple déposée par pulvérisation, évaporation ou dépôt chimique, afin d'éviter une oxydation du cuivre à long terme. L'inducteur 12 est avantageusement réalisé par une technique de micro-usinage ou par une technique de micro-fabrication. Dans le cas où l'inducteur 12 est réalisé par une technique de micro-usinage, l'inducteur 12 est avantageusement réalisé par micro-usinage laser dans une couche conductrice de quelques $\mu$m à quelques dizaines de $\mu$m d'épaisseur, et préférentiellement de 1 $\mu$m à 20 $\mu$m d'épaisseur, la couche conductrice étant par exemple déposée sur le substrat 11-a par électrodéposition. Dans le cas où l'inducteur 12 est réalisé par une technique de micro-fabrication, l'inducteur 12 est avantageusement réalisé par gravure chimique ou sèche d'une couche conductrice déposée sur le substrat 11-a par évaporation, par pulvérisation cathodique ou par électrodéposition, à travers un masque de résine obtenu par un procédé lithographique afin d'obtenir une résolution spatiale de l'inducteur 12 de l'ordre du $\mu$m. Il est également possible de réaliser l'inducteur 12 par un procédé d'électrodéposition à travers un masque de résine. Par rapport au micro-usinage, la micro-fabrication permet l'obtention de motifs plus précis et de plus petite taille pour l'inducteur 12, ce qui est avantageux pour intégrer un plus grand nombre d'inducteurs à la surface du substrat 11-a. L'épaisseur et la largeur de l'inducteur 12 sont choisies afin de permettre le passage du courant nécessaire à la génération d'un champ magnétique permettant l'excitation d'une zone de neurones, tout en évitant tout dommage pour les tissus environnants. L'inducteur 12 a typiquement une dimension latérale d'environ 500 $\mu$m.

La couche isolante 11-b qui est déposée sur l'inducteur 12 présente une épaisseur qui est choisie en fonction de la constante diélectrique du matériau de la couche isolante 11-b, pour permettre de limiter fortement tout couplage capacitif de l'inducteur 12 avec des tissus. Le matériau de la couche isolante 11-b est choisi biologiquement compatible, afin d'éviter une réaction inflammatoire des tissus. Une couche isolante 11-b réalisée en une bicouche alumine/nitrure de silicium de 1 $\mu$m d'épaisseur permet par exemple de réduire fortement tout couplage capacitif tout en assurant une tenue à long terme de l'explant scléral 11. Plus généralement, l'épaisseur de la couche isolante 11-b est typiquement comprise entre 1 $\mu$m et 20 $\mu$m. La couche isolante 11-b peut être un film de polymère. La couche isolante 11-b peut être un film d'oxyde de type $Al_2O_3$, $SiO_2$, $Si_3N_4$ ou une combinaison de ces matériaux.

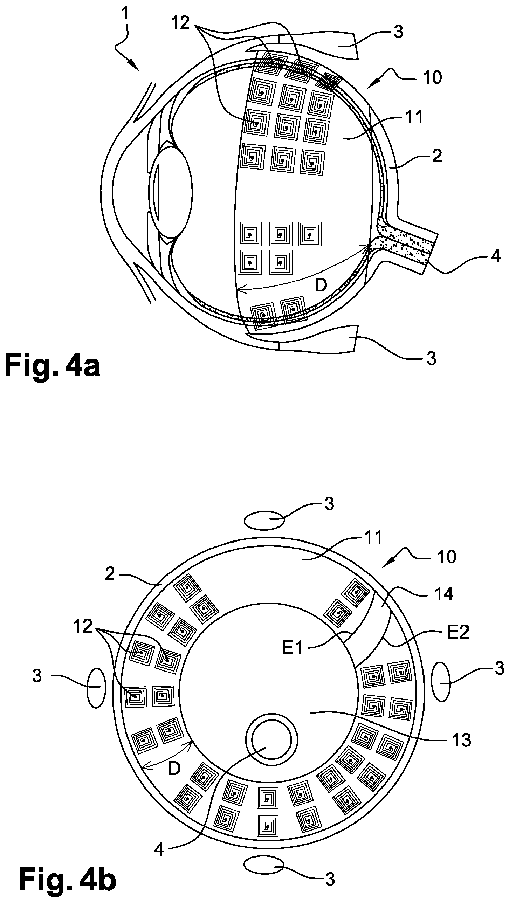

La figure 4a montre schématiquement une première vue du dispositif 10 de réhabilitation prothétique de la vision selon le premier mode de réalisation de l'invention, agencé au contact d'une partie de la sclère 2 d'un œil 1. La figure 4b montre schématiquement une deuxième vue du dispositif 10 de réhabilitation prothétique de la vision selon le premier mode de réalisation de l'invention, agencé au contact d'une partie de la sclère 2 de l'œil 1. Les figures 4a et 4b sont décrites conjointement.

Les figures 4a et 4b montrent l'œil 1, la sclère 2, les muscles extra-oculaires 3 et le nerf optique 4. Le dispositif 10 de réhabilitation prothétique de la vision est agencé au contact d'une partie de la sclère 2 de l'œil 1, l'explant scléral 11 ayant une forme de lentille, ou calotte sphérique, dont la courbure est adaptée à la courbure de la sclère 2. L'ouverture 13 de l'explant scléral permet le passage du nerf optique 4. La figure 4b montre en particulier le système d'accroché 14 de l'explant scléral 11, selon la variante du premier mode de réalisation de l'invention. Le système d'accroché 14 peut par exemple être réalisé grâce à un système de boucles, de berceaux, de manchons ou de clips, et préférentiellement grâce à un système de clips ou de berceaux compte-tenu des dimensions typiques de l'explant scléral 11.

Un mode d'implantation chirurgicale de l'explant scléral 11 du dispositif 10 de réhabilitation prothétique de la vision selon le premier mode de réalisation de l'invention est à présent décrit. Sous anesthésie locorégionale ou générale, désinsertion conjonctivale par péritomie au fornix, mise en place de fils de traction sur les quatre muscles oculomoteurs droits, exposition de la surface sclérale dans les quatre quadrants inter-musculaires, passage de l'explant scléral 11 sous les muscles droits et suture de l'explant scléral 11 à la sclère par des points épiscléraux non résorbables afin d'assurer la stabilité de l'explant scléral 11, puis fermeture de la conjonctive par points limbiques au fil résorbant. Cet exemple de mode d'implantation chirurgicale ne comporte pas d'étape d'ouverture de la paroi oculaire et élimine donc tout risque infectieux endo-oculaire. L'explant scléral 11 étant destiné à être agencé au contact de la sclère, et non au contact direct de la rétine, les risques de traumatisme ou de décollement rétinien sont évités. L'implantation chirurgicale de l'explant scléral selon un aspect de l'invention peut donc être réalisée au moyen d'une chirurgie courte, c'est-à-dire de moins d'une heure, pouvant avoir lieu en ambulatoire. A titre de comparaison, une implantation chirurgicale épi-rétinienne nécessite une intervention d'environ 3h, et une implantation chirurgicale sous-rétinienne nécessite quant à elle une intervention de plus de 6h, avec hospitalisation. Le dispositif 10 de réhabilitation prothétique de la vision selon le premier mode de réalisation de l'invention permet donc avantageusement une chirurgie d'implantation bien moins invasive, bien moins risquée et nettement plus courte que les chirurgies d'implantation des dispositifs en épi-rétinien ou en sous-rétinien selon l'état de la technique.

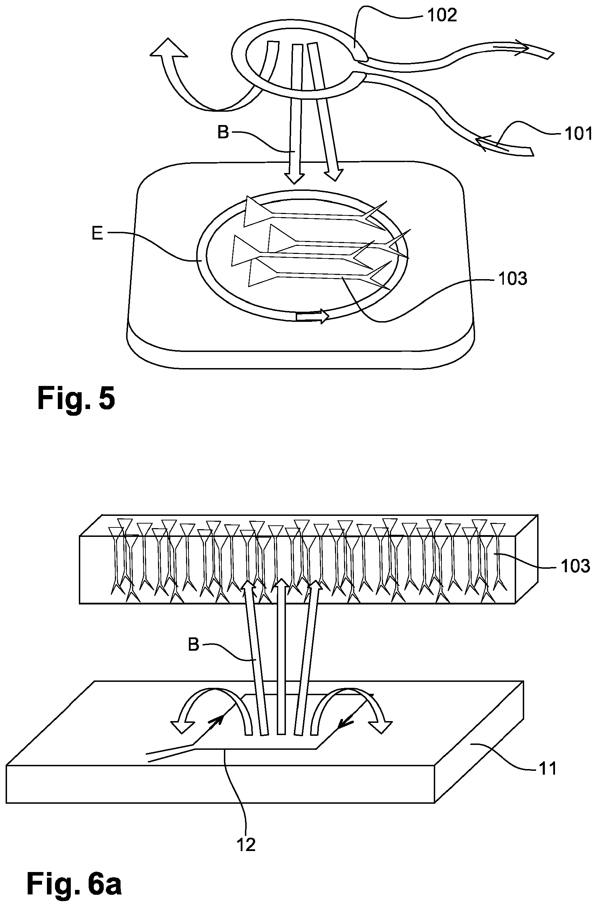

La figure 5 présente le principe physique d'activation neuronale d'au moins un neurone d'une zone cible par une impulsion électromagnétique sans contact, principe physique qui est appliqué par la présente invention. Un signal électromagnétique est créé par le passage d'une impulsion de courant 101 dans un conducteur 102, ou inducteur. L'impulsion de courant 101 est intense et de courte durée. Le conducteur 102 est par exemple obtenu par bobinage physique ou par des moyens de microfabrication, tels qu'une gravure ou un dépôt de couches minces. Le conducteur 102 est isolé galvaniquement de son environnement par une gaine isolante ou un film isolant. Dans le présent document, on entend par « le conducteur 102 est isolé galvaniquement de son environnement » le fait qu'il n'y a pas de contact électrique entre le conducteur 102 et son environnement.

Le signal électromagnétique créé sous l'effet du passage de l'impulsion de courant 101 dans le conducteur 102 présente une composante magnétique B qui dépend de l'amplitude de l'impulsion de courant 101 et de la géométrie du conducteur 102. L'amplitude du signal électromagnétique décroît typiquement comme l'inverse de la distance parcourue par ledit signal électromagnétique. Dans un milieu conducteur tel que celui des tissus, la variation de champ magnétique B est associée à un champ électrique E, qui se propage dans ledit milieu conducteur. Le champ électrique E agit alors sur au moins un neurone 103 en entraînant une activation dudit au moins un neurone 103 par dépolarisation transmembranaire. L'activation ou l'inhibition d'au moins un neurone 103 est notamment conditionnée par :

- la valeur du champ B et la variation du champ B dans le temps, afin d'atteindre une valeur seuil d'activation neuronale,
- l'orientation de l'impulsion de courant 101 par rapport à l'axe du au moins un neurone 103 à activer,
- la répartition spatiale du champ E créé, et en particulier le gradient du champ E créé, qui est un paramètre important de la dépolarisation ou hyperpolarisation membranaire entraînant activation ou inhibition du neurone.

Ainsi, dans le cas idéal où la composante du champ électrique E le long d'un composant neuronal, tel qu'une dendrite ou un axone, parfaitement droit présente un gradient positif, la membrane du neurone s'hyperpolarise, tandis qu'un gradient négatif, la membrane du neurone se dépolarise. Cependant, dans les tissus, les neurones présentent des morphologies non idéales et l'application d'un champ magnétique B variable d'intensité suffisante permet une activation des tissus neuronaux pour des orientations différentes du champ appliqué.

Plus généralement, l'intensité du champ magnétique requise pour l'activation d'un neurone par un inducteur varie avec la distance séparant l'inducteur du neurone. Ainsi, en stimulation magnétique transcranienne, la distance séparant l'inducteur du neurone est de l'ordre de 1 à 6 cm et les champs magnétiques délivrés correspondent à des champs électriques E seuil d'environ 100V/m. Pour une stimulation locale à environ 1 mm de distance et moins, les seuils attendus sont alors autour de 5V/m. Il est donc avantageux de rapprocher le ou les inducteurs du ou des neurones à exciter afin d'atteindre le seuil d'activation avec un courant ne générant pas d'échauffement incompatible avec l'implantation du ou des inducteurs. Une bobine planaire comprenant une seule spire peut être utilisée, mais afin d'augmenter la valeur du champ magnétique généré, il est avantageux d'augmenter le nombre de spires de la bobine, soit en créant des spires concentriques, soit en déposant deux spires de part et d'autre d'un substrat et en les connectant. Les deux techniques peuvent être combinées. Le dessin de la spire est choisi en fonction des dimensions recherchées pour l'inducteur et de la largeur des pistes, en veillant à minimiser la résistance de l'inducteur à des valeurs de l'ordre de quelques Ohms. Ainsi, pour une bobine de 400 $\mu$m de rayon extérieur fabriquée dans une couche de cuivre de 10 $\mu$m, une série de 10 spires concentriques de largeur unitaire 10 $\mu$m conduira typiquement à une résistance d'environ 3 Ohms et délivrera un champ B à 600 $\mu$m de 1,5mT pour 1A de courant. Une bobine de 400 $\mu$m de rayon extérieur et de 20 tours génèrera quant à elle un champ de 2,8 mT à 600 $\mu$m de distance.

Deux bobines de 400 $\mu$m de rayon extérieur, présentant chacune 5 spires concentriques de largeur unitaire 20 $\mu$m, et déposées sur les deux faces d'un substrat par exemple en kapton de 20 $\mu$m d'épaisseur, délivreront également environ 1,5 mT à 600 $\mu$m. D'une manière générale, les inducteurs sont dessinés suivant un motif permettant de délivrer une impulsion de champ magnétique B créant un champ électrique E suffisant dans la zone cible des neurones ganglionnaires à exciter, c'est-à-dire à une distance de 600 $\mu$m à 800 $\mu$m desdits inducteurs dans le cas d'un dispositif destiné à être implanté en épiscléral selon un aspect de l'invention. Pour une excitation souhaitée à une hauteur h d'une bobine planaire circulaire, le rayon externe de ladite bobine planaire circulaire générant l'impulsion électromagnétique sera typiquement d'environ h*√2, soit environ 850 $\mu$m pour une excitation à 600 $\mu$m et environ 1100 $\mu$m pour une excitation à 800 $\mu$m.

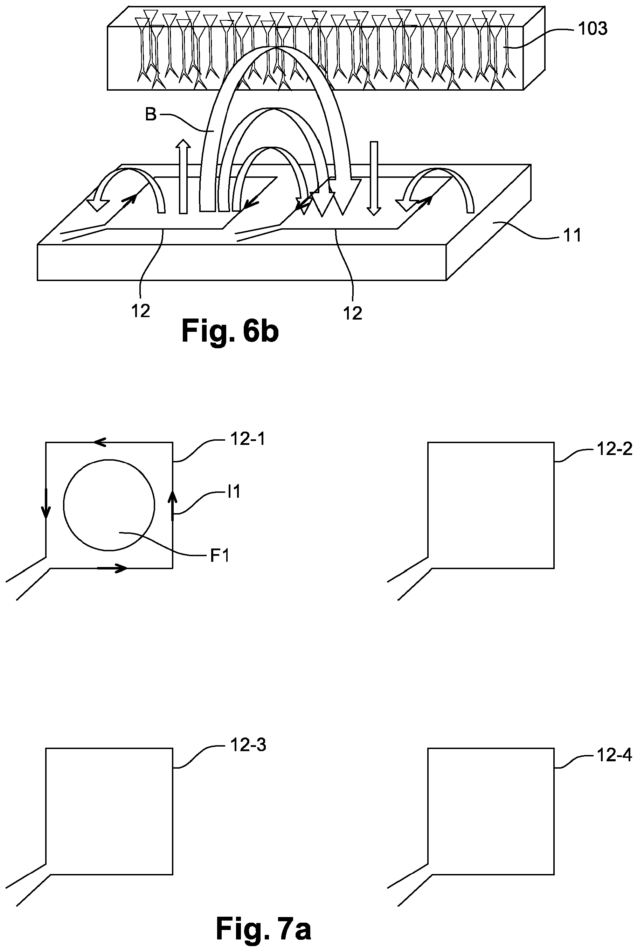

La figure 6a montre schématiquement le fonctionnement d'un dispositif de réhabilitation prothétique de la vision selon un aspect de l'invention, comportant un unique inducteur. La figure 6b montre schématiquement le fonctionnement d'un dispositif de réhabilitation prothétique de la vision selon un aspect de l'invention, comportant des premier et deuxième inducteurs.

La figure 6a montre un explant scléral 11 sur lequel est agencé un unique inducteur 12 qui est une boucle simple spire. Une impulsion de courant qui circule dans cet unique inducteur 12 à simple spire crée un champ magnétique B permettant d'activer des neurones 103 typiquement situés dans la demi-sphère au-dessus de la spire 12, le champ magnétique B étant maximal au milieu de la spire 12.

La figure 6b montre un explant scléral 11 sur lequel sont agencés deux inducteurs 12, chaque inducteur 12 étant une boucle simple spire. Les deux inducteurs 12 sont agencés dans un même plan, à la surface du substrat ou support de l'explant scléral 11, et sont alimentés par des courants de sens opposés. Le champ magnétique B créé par la circulation de ces courants de sens opposés est alors maximal entre les deux spires 12.

Le nombre et la disposition des inducteurs 12 permettent donc de définir précisément la zone qui sera excitée.

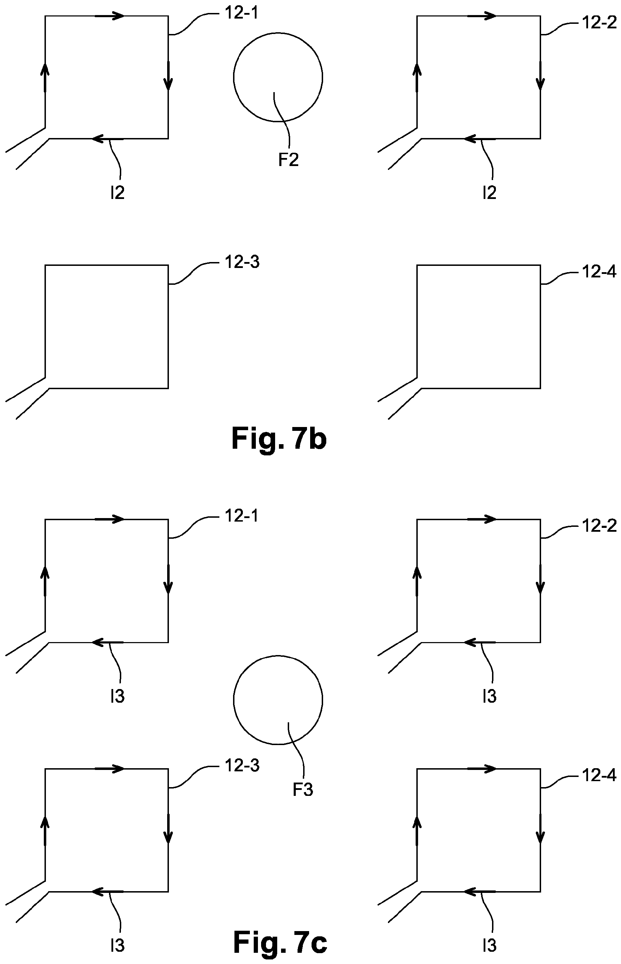

Les figures 7a, 7b et 7c illustrent trois configurations d'une même matrice comportant un premier inducteur 12-1, un deuxième inducteur 12-2, un troisième inducteur 12-3 et un quatrième inducteur 12-4, et le point focal de champ magnétique obtenu pour chaque configuration. Les premier, deuxième, troisième et quatrième inducteurs 12-1 à 12-4 sont agencés en une matrice carrée. Chacun des premier, deuxième, troisième et quatrième inducteurs 12-1 à 12-4 est une boucle simple spire.

La figure 7a montre une première configuration dans laquelle un seul élément de la matrice est alimenté. A la figure 7a, un premier courant I1 circule dans le premier inducteur 12-1. Aucun courant ne circule dans les deuxième, troisième et quatrième inducteurs 12-2, 12-3 et 12-4. Le premier courant I1 est choisi pour permettre d'atteindre le seuil d'excitation neuronale. Dans cette première configuration, un point focal F1 de champ magnétique est obtenu au centre de la première spire 12-1. Le maximum de champ magnétique se trouve au centre du point focal F1 de champ magnétique, pointant perpendiculairement au plan des inducteurs, en direction du lecteur.

La figure 7b montre une deuxième configuration dans laquelle deux éléments de la matrice sont alimentés. A la figure 7b, un deuxième courant I2 inférieur au premier courant I1 circule dans le premier inducteur 12-1 et dans le deuxième inducteur 12-2. Aucun courant ne circule dans les troisième et quatrième inducteurs 12-3 et 12-4. Le deuxième courant I2 n'est pas suffisant pour que le seuil d'excitation neuronale soit atteint pour chacun des premier et deuxième inducteurs 12-1 et 12-2, considérés individuellement. En revanche, la somme des contributions du deuxième courant I2 circulant dans les premier et deuxième inducteurs 12-1 et 12-2 permet de créer un point focal F2 de champ magnétique atteignant le seuil d'excitation neuronale. Le point focal F2 de champ magnétique est obtenu entre le premier inducteur 12-1 et le deuxième inducteur 12-2.

La figure 7c montre une troisième configuration dans laquelle les quatre éléments de la matrice sont alimentés. A la figure 7c, un troisième courant I3 inférieur au deuxième courant I2 circule dans chacun des premier, deuxième, troisième et quatrième inducteurs 12-1 à 12-4. Le troisième courant I3 n'est pas suffisant pour que le seuil d'excitation neuronale soit atteint pour chaque paire d'inducteurs considérée individuellement, c'est-à-dire pour la paire du premier inducteur 12-1 et du deuxième inducteur 12-2, la paire du deuxième inducteur 12-2 et du quatrième inducteur 12-4, la paire du troisième inducteur 12-3 et du quatrième inducteur 12-4 et la paire du premier inducteur 12-1 et du troisième inducteur 12-3. En revanche, la somme des contributions du troisième courant I3 circulant dans les premier, deuxième, troisième et quatrième inducteurs 12-1 à 12-4 permet de créer un point focal F3 de champ magnétique atteignant le seuil d'excitation neuronale. Le point focal F3 de champ magnétique est obtenu au centre du réseau formé par les premier, deuxième, troisième et quatrième inducteurs 12-1 à 12-4.

Les figures 6a et 6b d'une part, et les figures 7a à 7c d'autre part illustrent donc bien que l'on peut choisir, en fonction des inducteurs activés, les zones d'activation neuronale, avec une spécificité spatiale supérieure à celle donnée uniquement par le dessin des spires d'un unique inducteur.

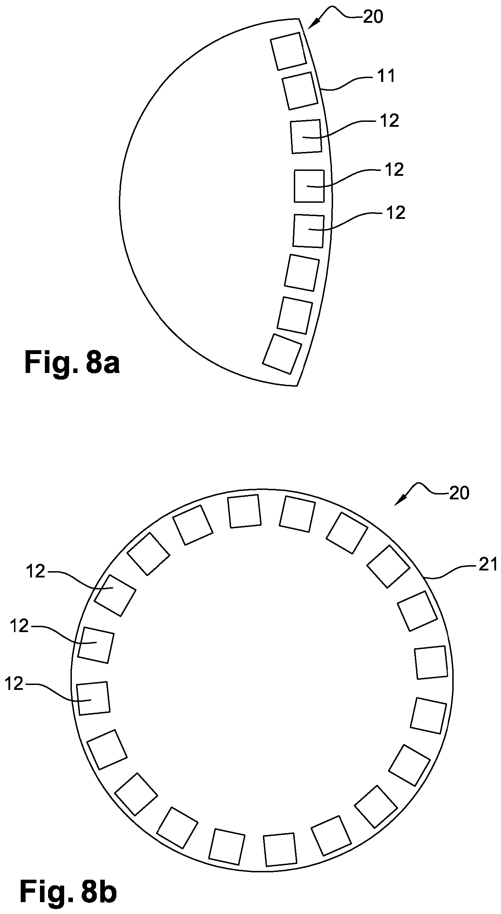

Un deuxième mode de réalisation de l'invention est à présent décrit. La figure 8a montre schématiquement une première vue, de côté, d'un dispositif 20 de réhabilitation prothétique de la vision selon le deuxième mode de réalisation de l'invention. La figure 8b montre schématiquement une deuxième vue, de face, du dispositif 20 de réhabilitation prothétique de la vision selon le deuxième mode de réalisation de l'invention. Les figures 8a et 8b sont décrites conjointement.

Le dispositif 20 de réhabilitation prothétique de la vision selon le deuxième mode de réalisation de l'invention comporte :

- un explant scléral ou lentille à support scléral ou lentille sclérale 21, dont la forme est adaptée pour être au contact d'au moins une partie de la sclère d'un œil, et
- une pluralité d'inducteurs 12 agencés sur l'explant scléral 21.

Selon le deuxième mode de réalisation de l'invention, l'explant scléral 21 est adapté pour être déposé devant l'œil, selon un mode d'insertion extra-oculaire ne nécessitant aucun acte chirurgical. Les inducteurs 12 sont agencés en périphérie de l'explant scléral 21 et sont destinés à agir sur des neurones ganglionnaires en périphérie externe de la rétine.

Dans l'exemple particulier illustré aux figures 8a et 8b, l'explant scléral 21 présente ainsi une forme de lentille dont la courbure est adaptée à la courbure naturelle de l'œil, et qui peut être déposée sur la sclère entourant la cornée. De manière analogue à l'explant scléral 11 précédemment décrit, l'explant scléral 21 peut être décrit comme étant une calotte sphérique, dont les dimensions sont choisies pour permettre l'adaptation de l'explant scléral 11 sur un œil.

De manière analogue à la description précédemment effectuée en lien avec la figure 3, l'explant scléral 21 comporte un substrat à la surface duquel au moins un inducteur 12 est agencé, et une couche isolante qui est déposée sur le au moins un inducteur 12 et la partie du substrat non recouverte par le au moins un inducteur 12. Des exemples de matériaux préférés pour le substrat, pour le au moins un inducteur et pour la couche isolante ainsi que des exemples de méthodes de fabrication ont précédemment été décrits en lien avec la figure 3.

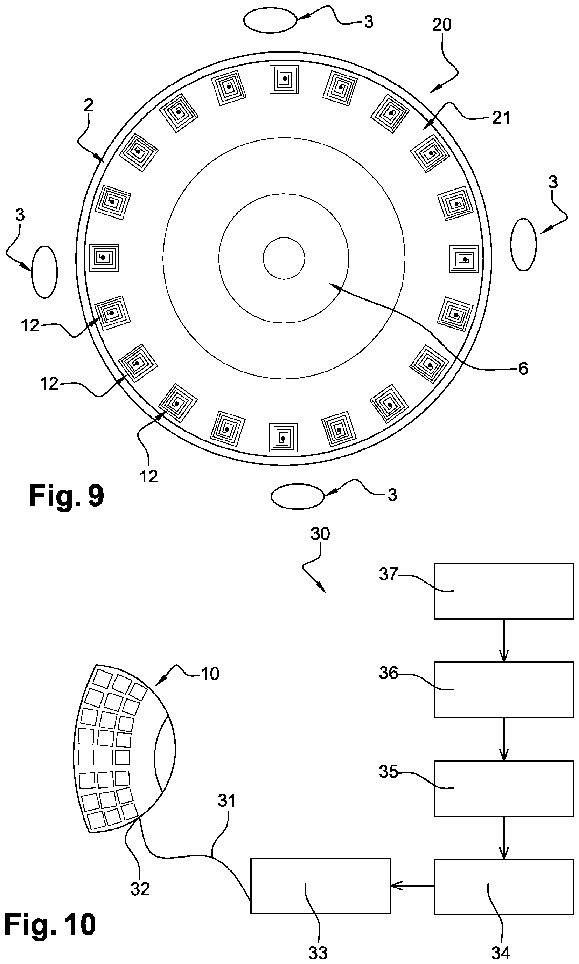

La figure 9 montre schématiquement une vue du dispositif 20 de réhabilitation prothétique de la vision selon le deuxième mode de réalisation de l'invention, agencé au contact d'une partie de la sclère 2 de l'œil 1. La figure 9 montre la sclère 2, les muscles extra-oculaires 3 et la cornée 6 de l'œil 1, l'explant scléral 21 ayant une forme de lentille, ou calotte sphérique, dont la courbure est adaptée à la courbure de la sclère 2.

La figure 10 montre une représentation schématique d'un système 30 de réhabilitation prothétique de la vision selon un aspect de l'invention, comportant le dispositif 10 selon le premier mode de réalisation de l'invention. Selon une alternative non représentée à la figure 10, le système 30 de réhabilitation prothétique de la vision peut comporter le dispositif 20 selon le deuxième mode de réalisation de l'invention, à la place du dispositif 10 selon le premier mode de réalisation de l'invention. La description qui suit est donc également valable pour un dispositif 10 selon le premier mode de réalisation de l'invention et pour un dispositif 20 selon le deuxième mode de réalisation de l'invention.

La figure 10 montre :

- un câble 31 comportant au moins un connecteur 32 d'un inducteur 12, chaque inducteur 12 ayant préférentiellement un connecteur 32, et le câble 31 comportant préférentiellement tous les connecteurs 32 des inducteurs 12;
- un multiplexeur 33 ;
- un générateur 34 d'impulsions de courant ;
- une unité de commande 35 du générateur d'impulsions 34 ;
- une unité de traitement d'images 36, et
- une unité d'acquisition d'images 37, ou caméra.

L'unité d'acquisition d'image 37 effectue l'acquisition d'une image et la transmet à l'unité de traitement d'images 36. L'unité de traitement d'images 36 effectue un codage de ladite image en valeurs numériques correspondant à des pixels simplifiés, en fonction du nombre d'inducteurs. Le codage de l'image est transmis à l'unité de commande 35. En fonction du codage reçu, l'unité de commande 35 envoie un signal de commande au générateur 34 d'impulsions de courant. Le générateur 34 envoie alors au moins une impulsion de courant vers le multiplexeur 33. Le multiplexeur 33 transmet alors la au moins une impulsion de courant à un inducteur ou à une pluralité d'inducteurs du dispositif 10 selon le premier mode de réalisation de l'invention ou du dispositif 20 selon le deuxième mode de réalisation de l'invention, via le câble 31. Les impulsions de courant envoyées dans les inducteurs 12 par le générateur 34 peuvent être de polarité positive ou négative. Chaque inducteur 12 est préférentiellement connecté au générateur 34 d'impulsions de courant par un connecteur 32, préférentiellement réalisé dans le même matériau que l'inducteur 12. Le dessin des connecteurs 32 est réalisé afin de minimiser les pertes dans la ligne de transmission de chaque impulsion, afin que le maximum de puissance délivrée soit dans l'inducteur 12. L'adaptation d'impédance est ainsi un paramètre important.

Chaque inducteur 12 peut être adressé individuellement. Alternativement, plusieurs inducteurs 12 peuvent être adressés, par exemple pour permettre l'obtention d'un point focal de champ magnétique avec un positionnement précis, ainsi qu'expliqué en lien avec les figures 6a, 6b et 7a à 7c. Dans le cas où plusieurs inducteurs 12 sont adressés, ou activés, l'adressage peut être réalisé en une seule fois ou bien en plusieurs séquences. Un adressage en plusieurs séquences permet notamment de limiter l'échauffement des inducteurs. En tenant compte d'un temps de montée dans l'inducteur, c'est-à-dire du temps nécessaire pour passer d'un courant nul à un courant de consigne dans l'inducteur, de l'ordre de 10 μs, et d'un temps de commutation d'un multiplexeur standard de l'ordre de quelques μs, il est possible d'activer séquentiellement de 1500 à 2500 d'inducteurs, un par un, sur une gamme de durées de 15 à 25 ms. Cette gamme de durées de 15 à 25 ms correspond à une gamme de fréquences de 40 à 60 Hz, c'est-à-dire la fréquence de fusion au-delà de laquelle l'œil ne perçoit pas distinctement deux signaux différents. La valeur de la fréquence de fusion dépend notamment de l'intensité de la modulation et de l'excentricité rétinienne. Dans le cas où des condensateurs sont utilisés, qui se déchargent lors de l'impulsion, le temps de charge de ces condensateurs, d'environ 100 μs, doit être pris en compte et il est alors possible d'activer séquentiellement de 130 à 220 inducteurs, un par un, en 15 à 25 ms.

Il est également possible d'utiliser des trains d'impulsions de courant d'intensité plus faible, mais permettant par effet de sommation d'atteindre un seuil d'excitation neuronale. Dans ce cas, la fréquence de répétition des trains d'impulsions doit être prise en compte pour calculer le temps total requis pour l'excitation individuelle de chaque inducteur, et pour calculer le nombre maximum d'inducteurs qu'il est possible d'exciter sur une durée de 15 à 25 ms. Un avantage offert par l'utilisation de trains d'impulsions de courant de faible intensité est la possibilité d'activer plusieurs inducteurs à la fois.

**Revendications**

1. Dispositif (10, 20) de réhabilitation prothétique de la vision **caractérisé en ce qu'**il comporte :

   - un explant scléral (11, 21) dont la forme est adaptée pour être au contact d'au moins une partie de la sclère (2) d'un œil (1), et

- au moins un inducteur (12) agencé sur l'explant scléral (11, 21) ; **caractérisé en ce que** l'explant scléral (11, 21) comporte un substrat (11-a) à la surface duquel est agencé l'inducteur (12), et une couche isolante (11-b) déposée sur l'inducteur (12) et sur la partie du substrat (11-a) qui n'est pas recouverte par l'inducteur (12) ; l'inducteur (12) créant un champ électromagnétique de stimulation d'une zone d'excitation et la couche isolante (11-b) isolant galvaniquement l'inducteur (12) de ladite zone d'excitation.

2. Dispositif (10, 20) de réhabilitation prothétique de la vision selon la revendication précédente **caractérisé en ce que** l'explant scléral (11, 21) présente une forme de calotte sphérique.

3. Dispositif (10, 20) de réhabilitation prothétique de la vision selon la revendication précédente **caractérisé en ce que** l'explant scléral (11, 21) comporte une partie centrale et une partie périphérique entourant la partie centrale, et **en ce que** le au moins un inducteur (12) est agencé sur la partie périphérique de l'explant scléral.

4. Dispositif (10) de réhabilitation prothétique de la vision selon l'une quelconque des revendications précédentes **caractérisé en ce que** l'explant scléral (11) comporte une ouverture (13).

5. Dispositif (10) de réhabilitation prothétique de la vision selon la revendication précédente **caractérisé en ce que** l'explant scléral (11) comporte une fente (14) agencée entre une périphérie extérieure de l'explant scléral et l'ouverture (13) de l'explant scléral, la fente (14) définissant deux extrémités (E1, E2) de l'explant scléral (11) pouvant être écartées l'une de l'autre.

6. Dispositif (10) de réhabilitation prothétique de la vision selon la revendication précédente **caractérisé en ce que** l'explant scléral (11) présente un système d'accroche (14) des deux extrémités (E1, E2) de l'explant scléral (11).

7. Dispositif (10, 20) de réhabilitation prothétique de la vision selon l'une quelconque des revendications précédentes **caractérisé en ce qu'**il comporte une pluralité d'inducteurs (12) agencée sur l'explant scléral (11, 21).

8. Système (30) de réhabilitation prothétique de la vision **caractérisé en ce qu'**il comporte :

- une unité d'acquisition d'images ;
- une unité de traitement d'images, recevant en entrée une image acquise par le système d'acquisition d'images et renvoyant en sortie un codage de ladite image ;
- une unité de commande, recevant en entrée le codage de ladite image et renvoyant en sortie une commande d'un générateur d'impulsions ;
- le générateur d'impulsions, envoyant selon la commande de l'unité de commande au moins une impulsion vers un multiplexeur ;
- le multiplexeur, transmettant la au moins une impulsion vers le au moins un inducteur ou vers au moins une partie de la pluralité d'inducteurs d'un dispositif (10, 20) de réhabilitation prothétique de la vision selon l'une quelconque des revendications précédentes ;
- le dispositif (10, 20) de réhabilitation prothétique de la vision selon l'une quelconque des revendications précédentes.

**Patentansprüche**

1. Vorrichtung (10, 20) zur prothetischen Wiederherstellung der Sicht, **dadurch gekennzeichnet, dass** sie umfasst:

- ein Sklera-Explantat (11, 21), dessen Form angepasst ist, um mit wenigstens einem Teil der Sklera (2) eines Auges (1) in Kontakt zu sein und;
- wenigstens einen Primärteil (12), der auf dem Sklera-Explantat (11, 21) angeordnet ist; **dadurch gekennzeichnet, dass** das Sklera-Explantat (11, 21) ein Substrat (11-a), an dessen Oberfläche der Primärteil (12) angeordnet ist, und eine isolierende Schicht (11-b), die auf dem Primärteil (12) und auf dem Teil des Substrats (11-a) aufgebracht ist, der nicht vom Primärteil (12) abgedeckt ist, umfasst; wobei der Primärteil (12) ein elektromagnetisches Stimulationsfeld eines Erregungsbereichs herstellt und die isolierende Schicht (11-b) den Primärteil (12) galvanisch von dem genannten Erregungsbereich isoliert.

2. Vorrichtung (10, 20) zur prothetischen Wiederherstellung der Sicht gemäß dem voranstehenden Anspruch, **dadurch gekennzeichnet, dass** das Sklera-Explantat (11, 21) eine sphärische Käppchenform aufweist.

3. Vorrichtung (10, 20) zur prothetischen Wiederherstellung der Sicht gemäß dem voranstehenden Anspruch, **dadurch gekennzeichnet, dass** das Sklera-Explantat (11, 21) einen zentralen Teil und einen umlaufenden Teil umfasst, der den zentralen Teil umgibt, und dass der wenigstens eine Primärteil (12) auf dem umlaufenden Teil des Sklera-Explantats angeordnet ist.

4. Vorrichtung (10, 20) zur prothetischen Wiederherstellung der Sicht gemäß irgendeinem der voranstehenden Ansprüche, **dadurch gekennzeichnet, dass** das Sklera-Explantat (11) eine Öffnung (13) umfasst.

5. Vorrichtung (10, 20) zur prothetischen Wiederherstellung der Sicht gemäß dem voranstehenden Anspruch, **dadurch gekennzeichnet, dass** das Sklera-Explantat (11) einen Schlitz (14) umfasst, der zwischen dem äußeren Umfang des Sklera-Explantats und der Öffnung (13) des Sklera-Explantats angeordnet ist, wobei der Schlitz (14) zwei Enden (E1, E2) des Sklera-Explantats (11) definiert, die voneinander beabstandet sein können.

6. Vorrichtung (10) zur prothetischen Wiederherstellung der Sicht gemäß dem voranstehenden Anspruch, **dadurch gekennzeichnet, dass** das Sklera-Explantat (11) ein Befestigungssystem (14) der zwei Enden (E1, E2) des Sklera-Explantats (11) aufweist.

7. Vorrichtung (10, 20) zur prothetischen Wiederherstellung der Sicht gemäß irgendeinem der voranstehenden Ansprüche, **dadurch gekennzeichnet, dass** sie eine Vielzahl von Primärteilen (12) umfasst, die auf dem Sklera-Explantat (11, 21) angeordnet sind.

8. System (30) zur prothetischen Wiederherstellung der Sicht, **dadurch gekennzeichnet, dass** es umfasst:

    - eine Erwerbseinheit von Bildern;
    - eine Verarbeitungseinheit von Bildern, die am Eingang ein Bild empfängt, das von dem Erwerbssystem von Bildern erworben ist und am Ausgang eine Kodierung des genannten Bildes zurückwirft;
    - eine Steuereinheit, die am Eingang die Kodierung des genannten Bildes empfängt und am Ausgang einen Befehl eines Impulsgenerators zurückwirft;
    - wobei der Impulsgenerator gemäß dem Befehl der Steuereinheit wenigstens einen Impuls zu einem Multiplexer sendet;
    - der Multiplexer, der den wenigstens einen Impuls an den wenigstens einen Primärteil oder an den wenigstens einen Teil der Vielzahl von Primärteilen einer Vorrichtung (10, 20) zur prothetischen Wiederherstellung der Sicht gemäß irgendeinem der voranstehenden Ansprüche überträgt;
    - die Vorrichtung (10, 20) der prothetischen Wiederherstellung der Sicht gemäß irgendeinem der voranstehenden Ansprüche.

**Claims**

1. Device (10, 20) for prosthetic vision rehabilitation **characterised in that** it comprises:

    - a scleral explant (11,21) with a shape that is suitable for being in contact with at least one portion of the sclera (2) of an eye (1), and
    - at least one inducer (12) arranged on the scleral explant (11, 21) **characterized in that** the scleral explant (11, 21) comprises a substrate (11 - a) on the surface of which the inducer (12) is arranged, and an insulating layer (11 -b) that is deposited on the inducer (12) and the portion of the substrate (11 -a) not covered by the inducer (12); the inducer (12) creating an electromagnetic field for stimulation of an excitation zone and the insulating layer (11-b) galvanically insulating the inducer (12) from said excitation zone.

2. Device (10, 20) for prosthetic vision rehabilitation according to the preceding claim **characterised in that** the scleral explant (11, 21) has a spherical calotte shape.

3. Device (10, 20) for prosthetic vision rehabilitation according to the preceding claim **characterised in that** the scleral explant (11, 21) comprises a central portion and a peripheral portion surrounding the central portion, and **in that** the at least one inducer (12) is arranged on the peripheral portion of the scleral explant.

4. Device (10) for prosthetic vision rehabilitation according to any of the preceding claims **characterised in that** the scleral explant (11) comprises an opening (13).

5. Device (10) for prosthetic vision rehabilitation according to the preceding claim **characterised in that** the scleral explant (11) comprises a slit (14) arranged between an external periphery of the scleral explant and the opening (13) of the scleral explant, the slit (14) defining two ends (E1, E2) of the scleral explant (11) being able to be moved apart from each other.

6. Device (10) for prosthetic vision rehabilitation according to the preceding claim **characterised in that** the scleral explant (11) has a system for attaching (14) the two ends (E1, E2) of the scleral explant (11).

7. Device (10, 20) for prosthetic vision rehabilitation according to any of the preceding claims **characterised in that** it comprises a plurality of inducers (12) arranged on the scleral explant (11, 21).

8. System (30) for prosthetic vision rehabilitation **characterised in that** it comprises:

- an image acquisition unit;

- an image processing unit, receiving as input an image acquired by the image acquisition system and sending back as output an encoding of said image;

- a control unit, receiving as input the encoding of said image and sending back as output a command of a pulse generator;

- the pulse generator, sending according to the command of the control unit at least one pulse to a multiplexer;

- the multiplexer, transmitting the at least one pulse to the at least one inducer or to at least one portion of the plurality of inducers of a device (10, 20) for prosthetic vision rehabilitation according to any of the preceding claims;

- the device (10, 20) for prosthetic vision rehabilitation according to any of the preceding claims.

**Fig. 1a**

EP 3 250 151 B1

**Fig. 1b**

**Fig. 1c**

14

**Fig. 2**

**Fig. 3**

**Fig. 4a**

**Fig. 4b**

**Fig. 5**

**Fig. 6a**

103

B

11

12

12

**Fig. 6b**

12-1

I1

F1

12-2

12-3

12-4

**Fig. 7a**

12-1

12-2

F2

I2

I2

12-3

12-4

**Fig. 7b**

12-1

12-2

I3

I3

F3

12-3

12-4

I3

I3

**Fig. 7c**

**Fig. 8a**

**Fig. 8b**

**Fig. 9**

**Fig. 10**

EP 3 250 151 B1

## RÉFÉRENCES CITÉES DANS LA DESCRIPTION

**Documents brevets cités dans la description**

- ES 2370014 A

**Littérature non-brevet citée dans la description**

- **E. BASHAM ; M. SIVAPRAKASAM ; W. LIU.** *Functional Magnetic Stimulation for Implantable Epiretinal Prosthesis,* 2005